# EUROPÄISCHE PATENTSCHRIFT

(11) **EP 1 234 388 B1**
(45) Veröffentlichungstag und Bekanntmachung des Hinweises auf die Patenterteilung: **03.03.2004**
(21) Anmeldenummer: 00989814.9
(22) Anmeldetag: 30.11.2000
(51) Int. Cl.: H04B 5/00, H04L 25/02

(54) **ANORDNUNG ZUR ÜBERTRAGUNG VON ELEKTRISCHEN SIGNALEN UND/ODER ENERGIE ZWISCHEN RELATIV ZUEINANDER DREHBAREN TEILEN**
ARRANGEMENT FOR TRANSMITTING ELECTRICAL SIGNALS AND/OR ENERGY BETWEEN PARTS THAT CAN BE ROTATED IN RELATION TO EACH OTHER
SYSTEME SERVANT A TRANSMETTRE DES SIGNAUX ET/OU DE L'ENERGIE ELECTRIQUES ENTRE DES ELEMENTS POUVANT TOURNER L'UN PAR RAPPORT A L'AUTRE

(30) Priorität: 30.11.1999 DE 19964130
(43) Veröffentlichungstag der Anmeldung: 28.08.2002
(73) Patentinhaber: Schleifring und Apparatebau GmbH, 82256 Fürstenfeldbruck (DE)
(72) Erfinder: LOHR, Georg, 82223 Eichenau (DE); SCHILLING, Harry, 91166 Georgensgmünd (DE)
(74) Vertreter: Lohr, Georg, Dr.
(86) Internationale Anmeldenummer: PCT/DE2000/004262
(87) Internationale Veröffentlichungsnummer: WO 2001/041315

(56) Entgegenhaltungen:
- EP-A- 0 429 261
- US-A- 5 140 696
- US-A- 5 892 411

## Beschreibung

### Technisches Gebiet

Die Erfindung bezieht sich auf eine Anordnung zur Übertragung elektrischer Signale und/oder Energie zwischen bewegten Teilen, die entlang einer beliebigen Trajektorie bzw. Bahn angeordnet sein können und miteinander in galvanischen oder zumindest kapazitiven bzw. induktiven Kontakt stehen.

### Stand der Technik

Elektrische Signale bzw. elektrische Energie müssen häufig zwischen relativ zueinander drehend beweglichen Teilen übertragen werden. Ein gängiges Verfahren dazu stellen Schleifbahnen und Schleifringe dar. Hier wird das Signal bzw. die Energie welche auf einem kreisförmig angeordneten Leiter zugeführt wird, mittels eines beweglichen Abgriffes abgeleitet. Die Anordnung muß nicht zwangsläufig koaxial sein, es ist auch eine gewisse Exzentrizität zulässig. Derartige Abgriffe können aus Kontaktfedern oder auch Kohlen bestehen, die einen guten galvanischen Kontakt ermöglichen. Ebenso ist es möglich, wie in der deutschen Patentanmeldung P 28 45 438 beschrieben, Signale bzw. Energie kapazitiv bzw. induktiv zu übertragen. In den nachstehenden Ausführungen wird der Klarheit halber anstelle der Begriffe "Signale bzw. Energie" nur noch auf den Begriff Signal bzw. Signalübertragung Bezug genommen. Weiterhin bezieht sich der Begriff Kanal auf einen kompletten Signalkanal, der in der Lage ist eine Information gleichzeitig zu übertragen und damit zumindest aus einem Hinleiter und einem Rückleiter besteht. Mehrere Kanäle können durchaus einen gemeinsamen Rückleiter besitzen. Wesentlich ist, daß ein Stromfluß zwischen der Signalquelle und der Last bzw. Signalsenke zustande kommt.

Die schmalbandige Signalübertragung zwischen drehend beweglichen Teilen entspricht dem Stand der Technik und stellt nur geringe Anforderungen an das Übertragungssystem. Eine breitbandige Signalübertragung hingegen stellt zusätzliche Ansprüche an die Übertragungstechnik. Hier sind grundsätzlich zwei Probleme zu lösen. Das erste Problem ist die Störstrahlung bzw. Einstrahlempfindlichkeit, das zweite Problem ist eine verzerrungsarme Signalübertragung.

Zur Verbesserung der Störabstrahlung bzw. Einstrahlempfindlichkeit sind verschiedene Maßnahmen bekannt. So werden in der US-Patentschrift 5,530,423 aufwendige Schirmmaßnahmen beschrieben, welche nur mit unvertretbar hohen Kosten hergestellt werden können. Weitaus wirtschaftlicher sind hier symmetrische Übertragungseinrichtungen wie beispielsweise in der PCT-Anmeldung PCT/EP95/01374 beschrieben. Die in dieser Schrift vorgestellte Lösung basiert auf idealen Streifenleitungen für die Signalübertragung. In der Praxis, insbesondere wenn mechanische Schleifbahnen eingesetzt werden, müssen zusätzliche Maßnahmen ergriffen werden. Weiterhin treten bei der hier beschriebenen Anordnung große Störpegel und erhebliche Übertragungsprobleme auf, wenn die Kontaktbahn (hier als Streifenleitung ausgeführt) als geschlossener Ring gestaltet wird. Eine solche Gestaltung als geschlossener Ring bietet jedoch große Vorteile in der Fertigung, da das Werkstück rotationssymmetrisch auf Drehbänken kostengünstig bearbeitet werden kann. Weiterhin ist ein auf mechanischen Kontakten basierendes Übertragungssystem ausschließlich mit geschlossenen Kontaktbahnen realisierbar, da ansonsten an der Trennstelle (Spalt) ein hoher Verschleiß des Kontaktmaterials auftreten würde.

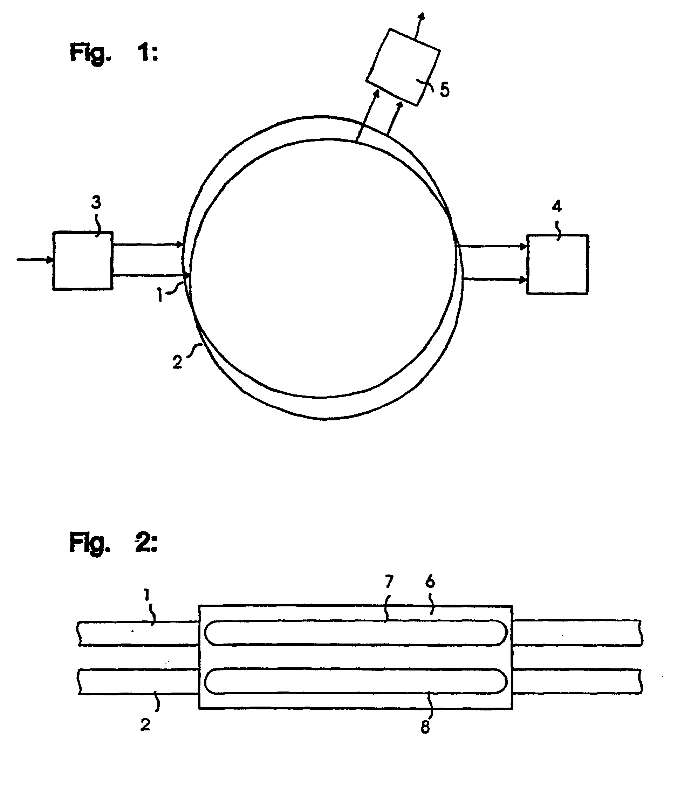

In der EP 0 429 261 A2 wird eine kontaktierende Kommunikationseinrichtung zwischen drehbar angeordneten Teilen, wie sie beispielsweise in Computertomographen eingesetzt werden kann beschrieben. Dabei ist eine Vorrichtung zur elektrischen Kommunikation zwischen gegeneinander drehbaren Systemen mittels Schleifkontakten angegeben. Das zu übertragende elektrische Signal wird mittels eines Senders (90) in eine Schleifbahn, welche mit einem Abschluss-Widerstand (140) abgeschlossen ist, eingespeist und mittels eines Schleifkontaktes (165g), welcher gegenüber der Schleifbahn beweglich angeordnet ist, abgegriffen und einem Empfänger (152) zur Weiterverarbeitung zugeführt. Weitere, parallel geführte Schleifbahnen (31f) und (31 h) sind mit den entsprechenden Bezugspotentialen (Schaltungsmasse) verbunden. Wie in Figur 1a dargestellt, wird das Leitersystem als Koaxialsystem betrachtet. In der praktischen Anwendung kann allerdings mit drei nebeneinander angeordneten Schleifbahnen keine ausreichende Schirmung erreicht werden, so dass die Anordnung einerseits sehr störempfindlich ist und andererseits sehr hohe Störpegel abstrahlt.

Die US 5,892,411 beschreibt eine Datenübertragungseinrichtung zur kontaktlosen Datenübertragung zwischen gegeneinander drehbaren Teilen. Die Signalübertragung erfolgt hier über eine Stripline, welche aus einer Leitung aus elektrisch leitfähigem Material und einer durch ein Isolationsmaterial von dieser getrennten elektrisch leitfähigen Basis besteht. Eine Streifenleitung (Stripline) zeichnet sich dadurch aus, dass zwischen dem Leiter und der leitfähigen Basis eine Verkopplung der elektrischen und magnetischen Felder besteht. So fließt entlang des Strompfads im Leiter durch die leitfähige Basis ein Strom entgegengesetzter Richtung zurück. Eine solche Anordnung zeichnet sich durch bessere Übertragungseigenschaften, verglichen mit der EP 0 429 261 A2, aus. Allerdings besteht auch hier noch eine hohe Störanfälligkeit und Störabstrahlung. Ein weiterer Nachteil ist die Auftrennung des Übertragungsweges entlang der Drehbewegung in vier unabhängige Stücke. Eine solche Aufteilung bereitet fertigungstechnische Probleme und im Falle einer kontaktierenden Übertragung mit Bürsten einen extrem hohen Abrieb an den Übergangsstellen.

### Darstellung der Erfindung

Der Erfindung liegt die Aufgabe zugrunde, eine Anordnung zur Übertragung elektrischer Signale und/oder Energie zwischen drehbar bewegten Teilen und einer entlang der Drehbewegung geschlossenen Kontaktbahn zu schaffen, die miteinander in galvanischem oder zumindest kapazitiven bzw. induktivem Kontakt stehen, derart weiterzubilden, daß eine störungsfreie und verzerrungsarme Signalübertragung bei Einhaltung der EMV-Grenzwerte gewährleistet werden kann.

Eine Lösung der Aufgabe ist im Anspruch 1 angegeben. Vorteilhafte Weiterbildungen sind Gegenstand der Unteransprüche.

Erfindungsgemäß ist eine Vorrichtung gemäß dem Oberbegriff des Anspruchs 1 derart ausgebildet, daß die elektrischen Leiter ein symmetrisch angeordnetes Leiter paar sind und der Sender das zu übertragende Signal symmetrisch in das Leiterpaar einspeist.

Das Abschlusselement ist gemäß dem Oberbegriff, dabei so ausgelegt, daß es das Leiterpaar weitgehend in dem zur Datenübertragung verwendeten Frequenzbereich reflexionsfrei abschließt. In der Regel wird dies ein Abschluß mit einem ohmschen Widerstand entsprechend dem Wellenwiderstand des Leiterpaares sein. In bestimmten Fällen kann

auch ein Abschluß aus einer Kombination von Blindelementen und Widerständen sinnvoll sein. Zum Empfang der Signale ist ein Empfänger vorgesehen, welcher die symmetrischen Signale des Senders auswertet. Der Empfänger ist beweglich gegenüber dem Sender angeordnet. Der Eingang des Empfängers muß derart hochohmig ausgeführt werden, daß er keine nennenswerten Reflexionen auf dem Leiterpaar verursacht. Die Einkoppelung des Signals vom Leiterpaar zum Empfänger kann über mechanische Schleifkontakte, entsprechend dem Stand der Technik, z. B. aus Silbergraphitkohlen oder auch Golddrähten bestehen. Eine besonders günstige Einkopplungsart ist ein kapazitiver Abgriff. Hier wird über die geringe Koppelkapazität zwischen dem Leiterpaar und einer kapazitiven sonde ein kleiner Anteil des Signals hochohmig und damit reflexionsfrei in den Empfänger ausgekoppelt.

In einer besonders günstigen Anordnung ist zumindest an einem der drehenden Teile ein Leiterpaar vorgesehen, an welchem der Sender und sein diametral gegenüber angeordneter Abschluß fest angeschlossen sind. Der Empfänger befindet sich dann an dem anderen drehbaren Teil und ist gegenüber dem Sender an unterschiedliche Positionen beweglich. Die Signaleinkopplung von dem Leiterpaar mit fest angekoppeltem Sender erfolgt nach dem Stand der Technik mit mechanischen Schleifkontakten bzw. induktiv oder kapazitiv zum Empfänger.

In einer weiteren vorteilhaften Ausführung der Erfindung ist zumindest an einem der beiden drehenden Teile ein Leiterpaar vorgesehen, das mit dem Empfänger fest verbunden ist. Der Sender und sein diametral gegenüber angeordnetes Abschlußelement sind hier entsprechend dem Stand der Technik über Schleifkontakte oder induktiv bzw. kapazitiv angekoppelt. Auch hier muß der Empfänger eine hohe Eingangsimpedanz besitzen, so daß er an dem Leiterpaar nur geringe Reflexionen verursacht. Der Sender und sein Abschlußelement sind zweckmäßigerweise über eine gemeinsame mechanische Struktur derart miteinander verbunden, daß unabhängig von der Drehbewegung die relative Position zueinander eingehalten wird.

In einer besonders vorteilhaften Ausgestaltung der Erfindung wird das Abschlußelement, welches diametral gegenüber dem Sender angeordnet ist, als ohmscher Widerstand ausgeführt.

In einer weiteren vorteilhaften Ausgestaltung der Erfindung wird der Widerstand des Abschlußelementes so dimensioniert, daß er dem Wellenwiderstand des Leiterpaares entspricht.

In einer besonders vorteilhaften Ausgestaltung der Erfindung ist im Sender ein zusätzliches Symmetrierglied vorhanden, welches dafür sorgt, daß vom Sender in das Leiterpaar ein hochsymmetrisches Signal eingespeist wird. Die Symmetrie dieses Signals steht in direktem Zusammenhang mit der Abstrahlung hochfrequenter Energie von dem Leiterpaar. Je symmetrischer das Signal ist, desto geringer ist die Abstrahlung und desto geringer sind die im Gerät nachweisbaren Störpegel.

In einer weiteren vorteilhaften Ausgestaltung der Erfindung befindet sich in der Eingangsbeschaltung des Empfängers ein Symmetrierglied, welches unsymmetrische Signalanteile am Empfängereingang unterdrückt und die gewünschten symmetrischen Anteile passieren läßt. Durch das Unterdrücken unsymmetrischer Anteile werden Gleichtaktstörungen, welche von externen Störquellen in das Leiterpaar eingekoppelt werden, unterdrückt. Die Wirkung des Symmetriergliedes hat einen unmittelbaren Einfluß auf die Störfestigkeit der Anordnung. Je höher die Unterdrückung unsymmetrischer Signalanteile ist, desto höher ist die Störfestigkeit der gesamten Anordnung.

In einer weiteren vorteilhaften Ausgestaltung der Erfindung enthält zumindest eines der Symmetrierglieder, welche im Sender bzw. Empfänger enthalten sein können, einen Transformator. Dieser Transformator ermöglicht gleichzeitig eine Potentialtrennung und ein gewisses Maß an Symmetrierung. Dazu muß die dem Leiterpaar zugeordnete Wicklung potentialfrei, d. h. an kein anderes festes Potential angebunden, sein.

In einer weiteren vorteilhaften Ausgestaltung der Erfindung befindet sich in zumindest einem der Symmetrierglieder, welche im Sender bzw. Empfänger enthalten sein können, ein Transformator, dessen, dem Leiterpaar zugeordnete Wicklung einen zusätzlichen Mittelabgriff besitzt. Dieser Mittelabgriff ist fest mit dem Massepotential der Anordnung verbunden. Dadurch läßt sich ein fester symmetrischer Bezug auf das Massepotential der Schaltung herstellen.

In einer weiteren vorteilhaften Anordnung enthält zumindest eines der Symmetrierglieder einen gleichstromgekoppelten Symmetrierübertrager. Derartige Symmetrierübertrager besitzen zwei auf den gleichen Kern gewickelte Wicklungen, die derart beschaltet sind, daß sich im Falle eines symmetrischen Stromflusses durch den Übertrager die Magnetfelder im Kern aufheben. Damit besitzt der Übertrager für symmetrische Signale eine äußerst niedrige Induktivität und damit eine niedrige Inpedanz. Im Falle von unsymmetrischen Signalen besitzt ein solcher Übertrager eine hohe Induktivität und damit eine hohe Inpedanz.

In einer weiteren vorteilhaften Anordnung ist zumindest eines der Symmetrierglieder, welche im Sender- bzw. Empfänger vorhanden sein können, derart ausgelegt, daß es sowohl einen Transformator zur Potentialtrennung als auch einen gleichstromgekoppelten Symmetrierübertrager enthält. Durch diese Kombination aus den beiden symmetrierenden Elementen ergibt sich eine wesentlich breitbandigere und höhere Symmetrierwirkung des Signals.

In einer weiteren vorteilhaften Ausgestaltung der Erfindung sind sämtliche Teile, welche das elektrische bzw. magnetische Feld des Leiterpaares beeinflussen können, innerhalb eines Abstandes vom doppelten der Breite des Leiterpaares hochsymmetrisch ausgeführt. Dies betrifft damit alle metallischen, dielektrischen und ferromagnetischen Teile. Durch diese symmetrische Anordnung können auch Verzerrungen im symmetrischen elektromagnetischen Feld des Leiterpaares vermieden werden und damit negative Einflüsse auf das Abstrahlverhalten bzw. die Störfestigkeit der ganzen Anordnung verringert werden.

In einer weiteren vorteilhaften Ausgestaltung der Erfindung wird die Verkoppelung der einzelnen Leiter des Leiterpaares zueinander vergrößert. Dies kann durch Einfügen von dielektrischem Material zwischen den Leitern erreicht werden. Im Falle des Aufbaus des Kontaktsystems auf einen Kunststoffträger kann dies durch zusätzliches Kunststoffmaterial zwischen den beiden Leitern erreicht werden. Hierbei sollte das Kunststoffmaterial idealerweise eine möglichst hohe Dielektrizitätskonstante besitzen.

In einer weiteren vorteilhaften Ausgestaltung der Erfindung wird die Verkoppelung des Leiterpaares mit benachbarten Leitern reduziert, indem außerhalb des Leiterpaares zusätzliche Luftspalte in den Kunststoffträger eingebracht werden. Damit verringert sich die Kapazität dieser Verkoppelung zu den benachbarten Leitern.

In einer weiteren Ausgestaltung der Erfindung sind zusätzlich an beiden Seiten des Leiterpaares symmetrisch Massebahnen angeordnet. Durch diese symmetrische Anordnung von Massebahnen kann die Verkoppelung von Signalen der Leiter zu weiteren benachbarten Leitern reduziert werden.

In einer weiteren vorteilhaften Ausgestaltung der Erfindung, wird die gegenüber dem Leiterpaar bewegliche Koppeleinrichtung derart ausgeführt, daß die parasitären Kapazitäten innerhalb der Koppeleinrichtung möglichst gering sind. Dadurch können die durch das Einkoppelelement verursachten Reflexionen auf dem Leiterpaar weiter reduziert werden. Ein solcher kapazitätsarmer Aufbau ist insbesondere an der Anschlußstelle des Empfängers sinnvoll, da hier erfindungsgemäß ein hohes Inpedanzniveau erforderlich ist.

In einer weiteren vorteilhaften Ausgestaltung der Erfindung ist die Koppeleinrichtung zur beweglichen Ankoppelung an das Leiterpaar derart dimensioniert, daß die parasitären Induktivitäten in der Koppeleinrichtung minimiert sind. Dies kann durch eine möglichst kompakte Bauweise realisiert werden und ermöglicht eine problemlose breitbandige und störungsarme Signaleinkopplung.

In einer weiteren vorteilhaften Ausgestaltung der Erfindung ist die Koppeleinrichtung zur beweglichen Ankoppelung an das Leiterpaar in Leitungstechnik ausgelegt, so daß diese Ankoppeleinrichtung selbst einen definierten Wellenwiderstand besitzt, der dem halben Wellenwiderstand des Leiterpaares entspricht. Eine solche Anordnung ist insbesondere an den Punkten der Einkoppelung des Senders bzw. seines diametral gegenüberliegenden Abschlusses an die Leiterstruktur sinnvoll, da hier reflexionsfreie Ankopplungen realisiert werden können.

In einer weiteren vorteilhaften Ausgestaltung der Erfindung wird ein nicht vernachlässigbares parasitäres Blindelement durch ein entsprechend komplementäres Blindelement kompensiert (Breitbandkompensation). Das Verfahren zur Dimensionierung solcher Breitbandkompensationen ist im Meinke/Gundlach Taschenbuch der Hochfrequenztechnik, Springer Verlag 1968, Seite 205 angegeben.

Eine andere vorteilhafte Ausgestaltung der Erfindung enthält ein Filter in der Ausgangsbeschaltung des Senders. Dieses Filter verhindert, daß störende Signalanteile an das Leiterpaar übertragen werden. Damit kann der Störemissionspegel der gesamten Anordnung wesentlich verringert werden. In der einfachsten Variante ist das Filter lediglich ein Tiefpaß dessen Grenzfrequenz oberhalb der maximal benötigten Signalfrequenz liegt. Komplexere Ausführungen des Filters können auch mehrkreisige Bandfilter oder auch aktive Filter sein. Gerade auch, wenn digitale Signale mit diskreten Spannungspegeln übertragen werden sollen, haben sich Sender mit linearen Verstärkern und zwischengeschalteten Filtern bewährt.

In einer ebenso vorteilhaften Ausgestaltung der Erfindung enthält der Empfänger ein Filter zur Unterdrückung von Frequenzen, welche nicht in das Übertragungsband gehören. Damit läßt sich die Störfestigkeit der Anordnung wesentlich erhöhen. Auch hier können die Filter im einfachsten Fall aus einem Tiefpaß bestehen. Sie können aber ebenfalls mehrkreisige Bandfilter oder aber auch aktive Filter sein. Auch bei digitalen Signalen mit diskreten Spannungspegeln können lineare Verstärker mit zwischengeschalteten Filtern eingesetzt werden.

In einer weiteren vorteilhaften Ausgestaltung der Erfindung liegen die dem zur Signalübertragung eingesetzten Leiterpaar benachbarten Bahnen auf Massepotential. Damit wird das Übersprechen zu benachbarten Bahnen wesentlich verringert.

In einer weiteren Ausgestaltung der Erfindung ist ein Schirm um das zur Signalübertragung verwendete Leiterpaar vorgesehen, der das Leiterpaar symmetrisch zumindest teilweise umschließt.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen hinsichtlich der Offenbarung aller im Text nicht näher erläuterten erfindungsgemäßen Einzelheiten ausdrücklich verwiesen wird. Es zeigen:
- Fig. 1: ein erstes Ausführungsbeispiel einer erfindungsgemäßen Anordnung,
- Fig. 2: ein kapazitives Koppelelement zur kapazitiven Ein- und Auskoppelung der Signale,
- Fig. 3: ein Symmetrierglied,
- Fig. 4: eine Ausgestaltung, bei der ein zusätzliches Dielektrikum zwischen den beiden Leitern zur verbesserten Verkoppelung eingebracht ist,
- Fig. 5: eine Anordnung mit zusätzlichen Luftspalten zur Verringerung der Verkoppelung zwischen benachbarten Teilen,
- Fig. 6: eine Anordnung, bei der das zur Signalübertragung verwendete Leiterpaar symmetrisch von Massebahnen umschlossen ist,
- Fig. 7: eine Ausgestaltung einer teilgeschirmten Anordnung, und
- Fig. 8: einen bevorzugten Anschluß einer teilgeschirmten Anordnung.

### Darstellung von Ausführungsbeispielen

Figur 1 zeigt eine beispielhafte Ausführung einer erfindungsgemäßen Anordnung. Die Signalübertragung zwischen gegeneinander beweglichen Einheiten erfolgt mittels eines symmetrisch angeordneten Leiterpaares (1) und (2). Das zu übertragenende Signal wird von einem Sender (3) erzeugt und in dieses Leiterpaar eingespeist. An einer zum Sender diametral gegenüberliegenden Position befindet sich das Abschlußelement (4). Dieses sorgt für einen reflexionsfreien Abschluß des Leitungssystems. Der Signalabgriff erfolgt durch den Empfänger (5), welcher eine beliebige Position am Umfang des Leiterpaares bezüglich dem Sender (3) und seinem Abschlußelement (4) einnehmen kann. Der Sender (5) besitzt eine hochohmige Eingangsstufe, so daß er keine nennenswerten Reflexionen auf dem Leiterpaar verursacht. Die Ankoppelung von Senderabschluß und Empfänger kann wahlweise durch mechanische Kontaktierung des Leiterpaares, durch Schleifkontakte wie Federdrähte oder Kohlen, aber auch kapazitiv bzw. induktiv erfolgen. Auf die Funktion der Anordnung hat es grundsätzlich keinen Einfluß, ob Sender bzw. Abschlußelement fest mit dem Leiterpaar verbunden sind und der Empfänger beweglich angeordnet ist oder der Empfänger fest mit dem Leiterpaar verbunden ist und Sender sowie Abschlußelement beweglich dazu angeordnet sind.

Figur 2 zeigt beispielhaft ein einfaches kapazitives Koppelelement zur kapazitiven Ein- und Auskoppelung der Signale. Dieses kapazitive Koppelelement (6) besitzt zwei Leiter (7) und (8), welche in einem maximalen Abstand von einigen Millimetern zu den Leitern (1) und (2) des Leiterpaares bewegt werden. Die hier erreichbare Kapazität von einigen pF ist für eine hochfrequente Signalübertragung in der Regel ausreichend.

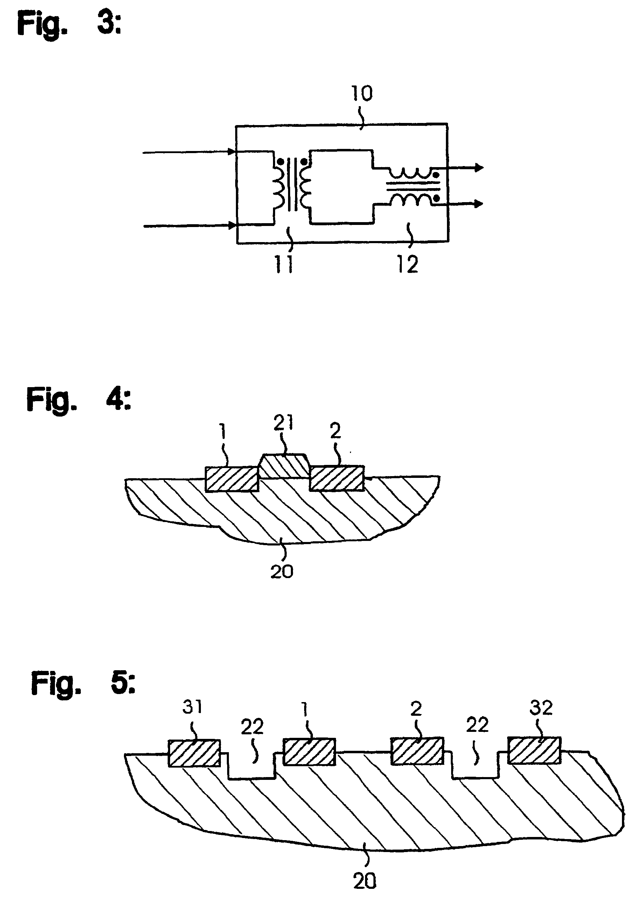

Figur 3 zeigt beispielhaft ein Symmetrierglied, welches sowohl im Sender als auch in dessen Abschlußelement bzw. im Empfänger eingesetzt werden kann. Das Symmetrierglied (10) besteht hier aus einem Transformator (11) zur Potentialtrennung der Signale sowie einem gleichstromgekoppelten Symmetrierübertrager (12). Selbstverständlich kann ein solcher Symmetrierübertrager auch als Guanella ausgeführt sein.

Figur 4 zeigt eine beispielhafte Ausgestaltung einer Anordnung bei der ein zusätzliches Dielektrikum zwischen den beiden Leitern zur verbesserten Verkoppelung eingebracht ist. Die beiden Leiter des Leiterpaares (1) und (2) sind auf dem Trägermaterial (20) befestigt. Zwischen den beiden Leiterbahnen befindet sich eine zusätzliche Materialanhäufung eines Dielektrikums (21), vorzugsweise des gleichen Materials wie das Trägermaterial. Damit wird die Kapazität der beiden Leiter zueinander und damit deren elektrische Verkoppelung vergrößert.

Figur 5 zeigt eine beispielhafte Anordnung mit zusätzlichen Luftspalten zur Verringerung der Verkoppelung zwischen benachbarten Teilen. Das Leiterpaar besteht hier aus dem ersten Leiter (1) und dem zweiten Leiter (2) sowie deren benachbarte Leiter (31) und (32). Um nun hier die Verkoppelung zwischen den benachbarten Leiterbahnen zu verringern, wird zusätzlich ein Luftspalt (22) jeweils zwischen den ersten Leiter (1) und seinem benachbarten Leiter (31) sowie den zweiten Leiter (2) und seinen benachbarten Leiter (32) eingebracht.

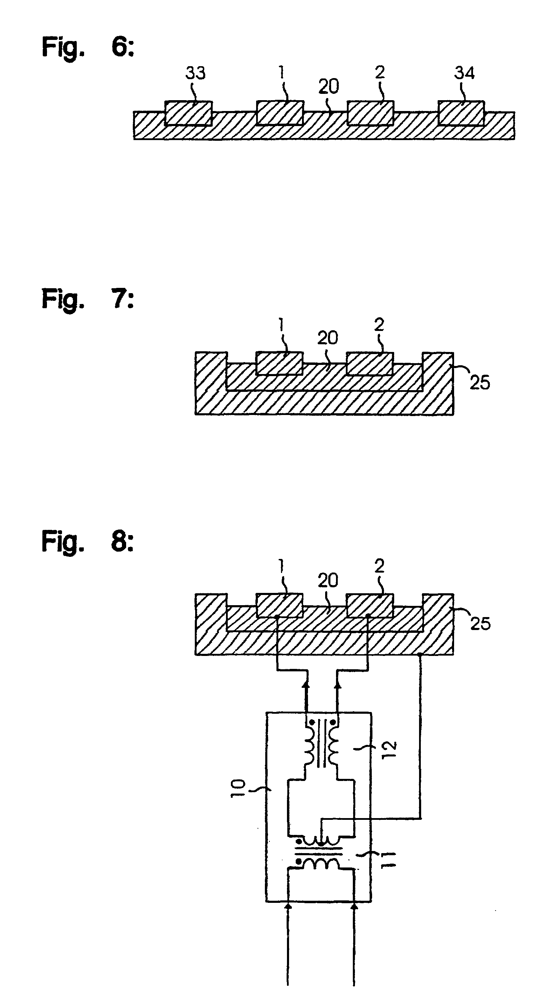

Figur 6 zeigt eine beispielhafte Ausgestaltung einer Anordnung bei der das zur Signalübertragung verwendete Leiterpaar (1) und (2) symmetrisch von Massebahnen (33) und (34) umschlossen sind. Dieses Massebahnen liegen auf einer definierten Schaltungsmasse. Die Kontaktierung kann puktförmig beispielsweise an der Einspeisestelle des Senders oder aber auch großflächig durch tragende Teile des Aufbaus oder durch Schirmstrukturen erfolgen.

Figur 7 zeigt eine beispielhafte Ausgestaltung einer teilgeschirmten Anordnung. Dabei sind die Leiter des zur Signalübertragung verwendeten Leiterpaares (1) und (2) symmetrisch zumindest teilweise vom Schirm (25) umschlossen. Die Isolation erfolgt hier durch ein Isolationsmaterial (20).

Figur 8 zeigt einen besonders günstigen Anschluß einer teilgeschirmten Anordnung. Die Signalzuführung erfolgt über ein Symmetrierglied, welches über einen Mittelabgriff des Transformators den Massebezug herstellt. Damit besitzt das Signal die höchstmögliche Symmetrie in Bezug auf die Schaltungsmasse. Diese Anschlußart ist selbstverständlich auch bei einer Anordnung mit zwei die Signalbahnen umgebenden Massebahnen möglich.

## Patentansprüche

1. Anordnung zur Übertragung von elektrischen Signalen und / oder Energie zwischen relativ zueinander drehbaren Teilen, von denen das Sendeteil (20), an dem der Sender (3) angeordnet ist, wenigstens zwei elektrische Leiter (1, 2) aufweist,
- deren Form der Trajektorie bzw. der Bahn der Bewegung angepasst ist,
- die eine entlang der Drehbewegung geschlossene Kontaktbahn darstellen und
- welche in dem Bereich, der dem Sender (3) diametral gegenüberliegt, weitgehend reflexionsfrei durch ein Abschlusselement (4) abgeschlossen sind, und
das andere Teil einen Empfänger (5) aufweist, welcher hochohmig ausgeführt ist und den Leitern (1, 2) des Sendeteils (20) angepasste Leiter (7, 8) aufweist, die nicht reflexionsfrei abgeschlossen sind, und mit den Leitern (1,2) an dem Sendeteil (20) galvanisch, induktiv und / oder kapazitiv gekoppelt sind,
**dadurch gekennzeichnet, dass** die elektrischen Leiter (1, 2) ein symmetrisch angeordnetes Leiterpaar sind und der Sender (3) das zu übertragende Signal symmetrisch in das Leiterpaar (1, 2) einspeist.

2. Anordnung nach Anspruch 1,
**dadurch gekennzeichnet, dass** der Empfänger (5) an beliebigen Positionen beweglich, das Signal mittels mechanischen Schleifkontakten oder induktiv bzw. kapazitiv abgreift.

3. Anordnung nach Anspruch 1 oder 2,
**dadurch gekennzeichnet, dass** der Eingang des Empfängers (5) derart hochohmig ausgeführt ist, dass er keine nennenswerten Reflexionen auf dem Leiterpaar (1, 2) verursacht.

4. Anordnung nach einem der Ansprüche 1 bis 3,
**dadurch gekennzeichnet, dass** der Empfänger (5) an das Leiterpaar (1, 2) fest angeschlossen ist und über Schleifkontakte oder induktiv bzw. kapazitiv angekoppelt der Sender (3) mit dem diametral gegenüber angeordneten Abschlusselement (4) auf einem gegenüber dem Leiterpaar (1, 2) beweglichen Träger vorgesehen ist.

5. Anordnung nach einem der Ansprüche 1 bis 4,
**dadurch gekennzeichnet, dass** das Abschlusselement (4) als Widerstand ausgeführt ist.

6. Anordnung nach einem der Ansprüche 1 bis 5,
**dadurch gekennzeichnet, dass** der Widerstand des Abschlusselements dem Wellenwiderstand des Leiterpaars entspricht.

7. Anordnung nach einem der Ansprüche 1 bis 6,
**dadurch gekennzeichnet, dass** zumindest im Sender (3) ein Symmetrierglied (10) vorhanden ist, welches für die Einspeisung eines hochsymmetrischen Signals in das Leiterpaar (1, 2) sorgt.

8. Anordnung nach einem der Ansprüche 1 bis 7,
**dadurch gekennzeichnet, dass** zumindest im Empfänger (5) ein Symmetrierglied (10) vorhanden ist, welches unsymmetrische Signalanteile am Empfängereingang unterdrückt und möglichst nur die symmetrischen Anteile passieren lässt.

9. Anordnung nach Anspruch 7 oder 8,
**dadurch gekennzeichnet, dass** zumindest eines der Symmetrierglieder (10) im Sender (3) bzw. Empfänger (5) einen Transformator (11) enthält, der gleichzeitig eine Potentialtrennung ermöglicht, und dessen dem Leiterpaar (1, 2) zugeordnete Wicklung keinem anderen festen Potential zugeordnet ist.

10. Anordnung nach einem der Ansprüche 7 bis 9,
**dadurch gekennzeichnet, dass** zumindest eines der Symmetrierglieder (10) im Sender (3) bzw. Empfänger (5) einen Transformator (11) enthält, der gleichzeitig eine Potentialtrennung ermöglicht und dessen dem Leiterpaar (1, 2) zugeordnete Wicklung über einen Mittelabgriff dem Massepotential zugeordnet ist.

11. Anordnung nach einem der Ansprüche 7 bis 10,
**dadurch gekennzeichnet, dass** zumindest eines der Symmetrierglieder (10) einen gleichstromgekoppelten Symmetrierübertrager (12) enthält, welcher aus zwei auf den gleichen Kern gewickelten Wicklungen besteht, die derart beschaltet werden, dass sich im Falle eines symmetrischen Stromflusses durch den Übertrager die Magnetfelder aufheben.

12. Anordnung nach einem der Ansprüche 7 bis 11,
**dadurch gekennzeichnet, dass** zumindest eines der Symmetrierglieder (10) sowohl einen Transformator (11) mit Potentialtrennung als auch einen gleichstromgekoppelten Symmetrierübertrager (12) enthält.

## Claims

1. Arrangement for transmitting electric signals and/or energy between parts which are rotatable relative to each other; a transmitting part (20) thereof, on which the transmitter (3) is disposed, having at least two electrical conductors (1, 2)
- which have a shape adapted to the trajectory or path of the movement,
- which represent a closed contact path along the rotational movement, and
- which are substantially terminated by a terminating element (4) in a reflection-free manner in the region located diametrically opposite to the transmitter (3);
and the other part thereof having a receiver (5) designed to be of high ohmic resistance and comprising conductors (7, 8) which are matched to the conductors (1, 2) of the transmitting part, and are not terminated in a reflexion-free manner, and are coupled galvanically, inductively and/or capacitively with the conductors (1, 2) on the transmitting part (20),
**characterized in that** the electrical conductors (1, 2) are a symmetrically disposed pair of conductors, and the transmitter (3) feeds the signal to be transmitted symmetrically into the pair of conductors (1, 2).

2. Arrangement according to claim 1,
**characterized in that** the receiver (5), adapted to be movable to desired positions, taps-off the signal by means of mechanical sliding contacts, or inductively, or capacitively.

3. Arrangement according to claim 1 or 2,
**characterized in that** the input of the receiver (5) is designed to be of such high ohmic resistance that it causes no appreciable reflections on the pair of conductors (1, 2).

4. Arrangement according to any one of claims 1 to 3,
**characterized in that** the receiver (5) is fixedly connected to the pair of conductors (1, 2); and that the transmitter (3), being inductively or capacitively coupled via sliding contacts, is provided together with the terminating element (4) which is disposed to be diametrically opposite, on a support which is movable relative to the pair of conductors (1, 2).

5. Arrangement according to any one of claims 1 to 4,
**characterized in that** the terminating element (4) is designed to be a resistor.

6. Arrangement according to any one of claims 1 to 5,
**characterized in that** the resistance of the terminating element corresponds to the wave resistance of the pair of conductors.

7. Arrangement according to any one of claims 1 to 6,
**characterized in that** a balancer (10) is provided at least in the transmitter (3) to ensure a feeding of a highly symmetric signal into the pair of conductors (1, 2).

8. Arrangement according to any one of claims 7 or 8,
**characterized in that** a balancer (10) is provided at least in the receiver (5) to suppress non-symmetric signal components at the receiver input, and to allow only the symmetric components to pass, as far as possible.

9. Arrangement according to claim 7 or 8,
**characterized in that** at least one of the balancers (10) in the transmitter (3) or receiver (5) includes a transformer (11) that simultaneously renders possible a separation of potential and has a winding, assigned to the pair of conductors (1, 2), which is not assigned to any other fixed potential.

10. Arrangement according to any one of claims 7 to 9,
**characterized in that** at least one of the balancers (10) in the transmitter (3) or receiver (5) includes a transformer (11) that simultaneously renders possible a separation of potential and has a winding, assigned to the pair of conductors (1, 2), which is assigned to ground potential via a centre tap.

11. Arrangement according to any one of claims 7 to 10,
**characterized in that** at least one of the balancers (10) contains a d.c. coupled balancing transformer (12) consisting of two windings which are wound onto the same core and connected so that in the case of a symmetric current flow through the transformer the magnetic fields cancel out.

12. Arrangement according to any one of claims 7 to 11,
**characterized in that** at least one of the balancers (10) includes a transformer (11) with separation of potential, and also a d.c. coupled balancing transformer (12).

## Revendications

1. Système servant à transmettre des signaux et/ou de l'énergie électriques entre des unités mobiles en rotation l'un par rapport à l'autre, dont l'unité transmettrice (20), à laquelle le transmetteur (3) est disposé, comprend au moins deux conducteurs électriques (1, 2)
- à une forme qui est adaptée au trajet ou respectivement à la voie du mouvement,
- qui représentent une voie de contact fermée le long du mouvement de rotation, et
- qui sont terminés, largement sans réflexions, par un élément terminal (4) dans la zone diamétralement opposée audit transmetteur (3), et
dont l'autre unité comprend un récepteur (5) à structure à haute impédance et des conducteurs (7, 8) adaptés auxdits conducteurs (1, 2) de ladite unité transmettrice (20), qui ne sont pas terminés sans réflexion et qui sont couplés auxdits conducteurs (1, 2) à ladite unité transmettrice (20) par voie galvanique, inductive et/ou capacitive,
**caractérisé en ce que** lesdits conducteurs électriques (1, 2) constituent une paire de conducteurs en arrangement symétrique, et **en ce que** ledit transmetteur (3) alimente, en symétrie, le signal à transmettre dans ladite paire de conducteurs (1, 2).

2. Système selon la revendication 1,
**caractérisé en ce que** ledit récepteur (5) est mobile aux toutes positions quelconques et prélève le signal moyennant des contacts à glissement ou par voie inductive ou capacitive.

3. Système selon la revendication 1 ou 2,
**caractérisé en ce que** l'entrée dudit récepteur (5) est une structure à impédance si haute qu'elle ne cause que des réflexions négligeables sur ladite paire de conducteurs (1, 2).

4. Système selon une quelconque des revendications 1 à 3,
**caractérisé en ce que** ledit récepteur (5) est raccordé, de manière fixe, à ladite paire de conducteurs (1, 2), et **en ce que** ledit transmetteur (3) avec ledit élément terminal diamétralement opposé, qui est couplé moyennant des contacts à glissement ou par voie inductive ou respectivement capacitive, est disposé à un support mobile par rapport ladite paire de conducteurs (1, 2).

5. Système selon une quelconque des revendications 1 à 4,
**caractérisé en ce que** ledit élément terminal (4) est réalisé sous forme d'une résistance.

6. Système selon une quelconque des revendications 1 à 5,
**caractérisé en ce que** la résistance dudit élément terminal correspond à l'impédance caractéristique de ladite paire de conducteurs.

7. Système selon une quelconque des revendications 1 à 6,
**caractérisé en ce qu'**un élément d'équilibrage (10) est disposé au moins dans ledit transmetteur (3), qui assure l'alimentation d'un signal à haute symétrie dans ladite paire de conducteurs (1, 2).

8. Système selon une quelconque des revendications 1 à 7,
**caractérisé en ce qu'**un élément d'équilibrage (10) est disposé au moins dans ledit récepteur (5), qui supprime des fractions de signal non symétriques à l'entrée dudit récepteur et ne laisse passer que des fractions symétriques si possible.

9. Système selon la revendication 7 ou 8,
**caractérisé en ce qu'**au moins un desdits éléments d'équilibrage (10) dans ledit transmetteur (3) ou respectivement ledit récepteur (5) comprend un transformateur (11), qui permet, en même temps, une séparation de potentiels et comprend une bobine, qui est affectée à ladite paire de conducteurs (1, 2) et qui n'est pas affecté à un autre potentiel invariable.

10. Système selon une quelconque des revendications 7 à 10,
**caractérisé en ce qu'**au moins un desdits éléments d'équilibrage (10) dans ledit transmetteur (3) ou respectivement ledit récepteur (5) comprend un transformateur (11), qui permet, en même temps, une séparation de potentiels et comprend une bobine, qui est affectée à ladite paire de conducteurs (1, 2) et qui est affectée, via une prise centrale, au potentiel de masse.

11. Système selon une quelconque des revendications 2 à 10,
**caractérisé en ce qu'**au moins un desdits éléments d'équilibrage (10) comprend un transformateur équilibreur (12) lié en courant continu, qui consiste de deux bobines enroulées sur le même noyau, qui sont filées d'une telle manière, que les champs magnétiques s'éliminent au cas d'un écoulement de courant en symétrie à travers ledit transformateur.

12. Système selon une quelconque des revendications 7 à 11,
**caractérisé en ce qu'**au moins un desdits éléments d'équilibrage (10) comprend un transformateur (11) à séparation des potentiels et aussi un transformateur équilibreur lié en courant continu (12).
